# EUROPEAN PATENT SPECIFICATION

(11) **EP 0 424 028 B1**
(45) Date of publication and mention of the grant of the patent: **27.09.1995**
(21) Application number: 90311150.8
(22) Date of filing: 11.10.1990
(51) Int. Cl.: C07C 57/30

(54) **S(+)-ibuprofen-L-amino acid and S(+)-ibuprofen-D-amino acid as onset hastened enhanced analgesics**
S(+)-Ibuprofen-L-Aminosäure und S(+)-Ibuprofen-D-Aminosäure als Analgetika mit grösserem pharmakologischem Potential
Acide S(+)-ibuprofène-L-amino et l'acide S(+)-ibuprofène-D-amino comme agents analgésiques puissants

(30) Priority: 17.10.1989 US 422466
(43) Date of publication of application: 24.04.1991
(73) Proprietor: Merck & Co., Inc., Rahway New Jersey 07065-0900 (US)
(72) Inventor: Kwan, King Chiu, Blue Bell, PA 19422 (US)
(74) Representative: Cole, William Gwyn

(56) References cited:
- EP-A- 0 267 321
- GB-A- 963 265
- GB-A- 1 471 910
- US-A- 4 851 444

## Description

### BACKGROUND OF THE INVENTION

Ibuprofen (I) is a well-known nonsteroidal antiinflammatory drug which possesses analgesic and antipyretic activity.

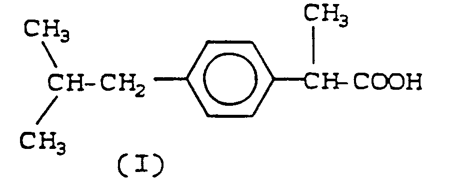

Ibuprofen has been marketed as a racemic mixture of the S(+) and R(-) enantiomers; however, recently there have been suggestions in the literature regarding an increased pharmaceutical benefit for S(+)-ibuprofen relative to the racemic mixture, see, for example, A. Sunshine et al U.S. Patent 4,851,444, D. Lowe et al EPO publication 267,321, K. Williams et al Brit.J. of Pharm. 35 3404 (1986).

Ibuprofen as a carboxylic acid can form salts with bases, such as basic amino acids, examples of which are lysine and arginine. The low solubility of the acid form of ibuprofen is overcome by the use of the lysinate salt of ibuprofen. U.S. Patent 4,279,926 describes the use of the lysine salt of Ibuprofen in relieving pain and inflammatory conditions in warm-blooded animals. However, there is no description in the literature of the compound S(+)-ibuprofen-L-lysine, i.e. the L-lysinate salt of S(+)-ibuprofen.

### DETAILED DESCRIPTION OF THE INVENTION

This invention relates to the crystalline lysine salt of S(+)-ibuprofen.

Where the particular stereoisomeric form is described in this invention, it is meant to be substantially free of any other stereoisomeric configuration. Substantially free should be taken to mean that the active ingredient contains at least 90% by weight of the desired stereoisomer and 10% or less of other stereoisomers. Preferably the weight % ratio is better than 95:5 and most preferably 99:1 or better. For example, S(+)-ibuprofen-L-lysine contains at least 90% by weight of this stereoisomer and 10% or less of other configurations of Ibuprofen-Lysine.

Crystalline S(+)-ibuprofen-L-lysine exhibits an onset-hastened, enhanced analgesic response of longer duration compared to ibuprofen. Specifically, applicants have found that in humans crystalline S(+)-ibuprofen-L-lysine has a faster onset of analgesic action, an enhanced analgesic response and a longer duration of action than the same dose of racemic ibuprofen in the acid form or as racemic ibuprofen lysinate. Crystalline S(+)-ibuprofen-L-lysine also exhibits a more rapid onset and an enhanced response of longer duration when compared to S(+)-ibuprofen.

The employment of a particular basic amino acid stereoisomeric salt of ibuprofen which contains only one enantiomeric form of the acid and one enantiomeric form of the basic amino acid avoids introducing into the human body enantiomeric forms which are less therapeutically effective, may be toxic and which may put an unnecessary burden on the metabolic systems.

An embodiment of this invention is a pharmaceutical composition for treating pain and inflammation in a human subject in need of such treatment, said composition comprising an onset-hastening, enhancing analgesically, effective amount of crystalline S(+)-ibuprofen-L-lysine and a non-toxic pharmaceutically acceptable carrier.

A further embodiment is the use in the manufacture of a medicament for treating pain and inflammation in a human subject in need of such treatment of an onset-hastening, enhancing analgesically, effective amount of crystalline S(+)-ibuprofen-L-lysine.

The amount of S(+)-ibuprofen-L-lysine to be administered will vary depending on the extent of the pain and/or inflammation and the tolerance of the patient. Typically the amounts will vary from 50mg to 800mg (amounts measured in mg ibuprofen). Preferred doses are 50, 100, 200, 300, 400, 600 and 800mg.

Compositions of S(+)-ibuprofen-L-lysine may be administered orally, parenterally, or topically. The compositions may contain inert carriers such as lactose, starch, calcium sulfate, mannitol and sucrose. Suitable binders, lubricants, flavoring agents, disintegrating agents and coloring agents may also be added.

The increased therapeutic benefits described above for crystalline S(+)-ibuprofen-L-lysine can be demonstrated by single oral dose randomized, double-blind studies comparing it to other stereoisomeric forms of ibuprofen and salts thereof in patients experiencing moderate or severe pain following oral surgery. Speed of onset and analgesic efficacy can be evaluated by having patients assess their pain intensity and degree of pain relief at 1/2, 1, 1-1/2, 2, 3, 4, 5 and 6 hours after dosing. Additionally, patients may use a stopwatch to measure the time until meaningful pain relief was experienced.

Other pain models include postoperative pain, postpartum uterine cramping pain, and dysmenorrhea. The design of all studies is randomized, double-blind and, in addition, the dysmenorrhea studies are a four-menstrual period crossover design. In all studies patients evaluate their degree of pain relief at time points beginning. 1/2 hour after dosing. A stopwatch may be used to measure time to meaningful pain relief. Sample sizes in the studies are sufficient to demonstrate the increased efficacy of crystalline S(+)-ibuprofen-L-lysine and to detect significant differences in speed of analgesic activity between crystalline S(+)-ibuprofen-L-lysine and other stereoisomeric forms of ibuprofen and salts thereof.

S(+)-ibuprofen may be prepared following the procedures described in U.S. Patent 4,851,444. Alternatively S(+)-ibuprofen may be prepared following the resolution method given in co-pending EP-A-0390273, the contents of which are hereby incorporated by reference. L-lysine is commercially available from the Sigma Chemical Company or the Aldrich Chemical Company, or S(+)-ibuprofen-L-amino salts and S(+)-ibuprofen-D-amino acid salts may be prepared following the procedure of U.S. Patent 4,279,926 but substituting the particular enantiomers for the racemic mixtures described in that patent.

The following examples illustrate the present invention and its incorporation into pharmaceutical compositions for therapy.

### EXAMPLE 1

### Typical Pharmaceutical Compositions Containing A Compound Of The Invention

A: Dry Filled Capsules Containing 200 mg of Ibuprofen Per Capsule.

| Ingredient | Amount Per Capsule (mg) |
|---|---|
| S (+)-Ibuprofen-L-Lysine | 342 mg |
| Alcohol SD 3A Anhydrous | - |
| Magnesium Stearate Impalpable Powder NF | 2.00 mg |
| Hard Gelatin Capsule #0 | 96.0 mg |
| | 440 mg |

B: Film Coated Tablet Containing 200 mg of Ibuprofen Per Capsule.

| Ingredient | Amount Per Tablet (mg) |
|---|---|
| S (+)-Ibuprofen-L-Lysine | 342 mg |
| Povidone USP | 17.0 mg |
| Avicel PH102 | 18.0 mg |
| Alcohol SD 3A Anhydrous | - |
| Water Purified | - |
| Magnesium Stearate Impalpable Powder NF | 4.00 mg |
| Core Tablet Weight | 381 mg |
| Hydroxypropyl Methylcellulose USP 6CPS | 4.00 mg |
| Hydroxypropyl Cellulose LF NF W/<0.3% Silica | 4.00 mg |
| Titanium Dioxide USP | 1.60 mg |
| Talc USP Purified | 0.40 mg |
| Coated Tablet Weight (Theoretical) Actual Weight (408 mg) Includes the 5% Hydration of Active Ingredient During Granulation | 391 mg |

C: Injectable Solution:

| Ingredient | Amount (mg) |
|---|---|
| S(+)-Ibuprofen-L-lysine* | 27.3 mg |
| Mannitol | 27.3 mg |
| Water for Injection | q.s. to 1.0 ml |

| | |
|---|---|
| *Equivalent to 16.0 mg of Ibuprofen free acid content. | |

The above formula may be diluted in Sodium Chloride Injection for slow infusion.
D: Suppository

| Ingredient | Amount (mg) |
|---|---|
| S(+)-Ibuprofen-L-lysine* | 1360 |
| Polyethylene glycol⁺ | 1631 |
| Tocopherol or BHA or BHT | 9 |

| | |
|---|---|
| *Equivalent to 800 mg of Ibuprofen free acid content. The drug in this form can be varied from 50 mg to 800 mg Ibuprofen and the necessary glycol or fatty vehicle modified to yield a suppository of about 3 to 3.2 grams. | |
| ⁺A waxy or fatty vehicle such as cocoa butter or Suppocire or Wipepsol can also be employed. | |

### EXAMPLE 2

### Preparation of S(+)-Ibuprofen-L-Lysine

A 500 ml 3 neck flask equipped with mechanical stirrer and thermometer was charged with H₂O (42 ml) and L-lysine (37.5 g, 0.25 mole). The mixture was agitated until a solution was obtained. A solution of S(+)-ibuprofen in absolute ethanol (52.6 g, 0.25 mole in 250 ml ethanol) was made up and added to the aqueous lysine over 5 minutes at a temperature ≦ 35°C. The resulting solution was filtered through a sintered glass funnel (medium frit) to remove any insoluble material. The filtrate was transferred to a 3000 ml 3-neck flask equipped with a mechanical stirrer, addition funnel and thermometer. To this clear solution was added 575 ml of ethanol over 5 minutes at 20-25°C. The turbid solution was seeded with the L-lysine salt of S(+)-ibuprofen (50 mg) and an additional 400 ml of ethanol was added over 10 minutes. The resulting suspension was aged at 20-25°C for 1 hour with agitation, cooled to 0-5°C and held for 1 hour. The product was filtered, washed with 5 ± 5°C ethanol (2 x 1 mmol) and dried in vacuo (50°C) to yield 74 g (84%) of the titled compound. [α]₄₀₅ = +14.9°C (C = 1, methanol).

## Claims

1. Crystalline S(+)-ibuprofen-L-lysine substantially free from any other ibuprofen-lysine stereoisomer.

2. The compound of Claim 1 wherein the weight ratio of S(+)-ibuprofen-L-lysine to other stereoisomers is at least 95:5.

3. The compound of Claim 1 wherein the weight ratio is at least 99:1.

4. A pharmaceutical composition which comprises a compound as claimed in any of claims 1 to 3 and a non-toxic pharmaceutically acceptable carrier.

5. The use of a compound as claimed in any of claims 1 to 3 in the manufacture of a medicament for treating pain and inflammation in a human.

## Patentansprüche

1. Kristallines S(+)-Ibuprofen-L-lysin, das im wesentlichen frei von irgendeinem anderen Ibuprofen-lysin-Stereoisomer ist.

2. Die Verbindung nach Anspruch 1, worin das Gewichtsverhältnis von S(+)-Ibuprofen-L-lysin zu anderen Stereoisomeren wenigstens 95:5 ist.

3. Die Verbindung nach Anspruch 1, worin das Gewichtsverhältnis wenigstens 99:1 ist.

4. Eine pharmazeutische Zusammensetzung, die eine wie in irgendeinem der Ansprüche 1 bis 3 beanspruchte Verbindung und einen nicht toxischen, pharmazeutisch annehmbaren Träger umfaßt.

5. Die Verwendung einer wie in irgendeinem der Ansprüche 1 bis 3 beanspruchten Verbindung für die Herstellung eines Medikaments zur Schmerz- und Endzündungsbehandlung bei einem Menschen.

## Revendications

1. S(+)-ibuprofène-L-lysine cristalline essentiellement exempte de tout autre stéréoisomère d'ibuprofène-lysine.

2. Compose selon la revendication 1, dans lequel le rapport pondéral de la S(+)-ibuprofène-L-lysine aux autres stéréoisomères est d'au moins 95:5.

3. Compose selon la revendication 1, dans lequel le rapport pondéral est d'au moins 99:1.

4. Composition pharmaceutique comprenant un composé selon l'une quelconque des revendications 1 à 3 et un véhicule pharmaceutiquement acceptable et non toxique.

5. Utilisation d'un composé selon l'une quelconque des revendications 1 à 3, pour la fabrication d'un médicament destiné au traitement de la douleur et de l'inflammation chez l'homme.
